# EUROPÄISCHE PATENTSCHRIFT

(11) **EP 0 882 709 B1**
(45) Veröffentlichungstag und Bekanntmachung des Hinweises auf die Patenterteilung: **25.07.2001**
(21) Anmeldenummer: 98109285.1
(22) Anmeldetag: 22.05.1998
(51) Int. Cl.: C07C 403/24, C07D 493/08

(54) **Verfahren zur Herstellung von Zeaxanthin, Zwischenprodukte für dieses Verfahren und Verfahren zu deren Herstellung**
Process for the preparation of zeaxanthin, intermediates for this process and process for their preparation
Procédé pour la préparation de la zéaxanthine, intermédiaires pour ce procédé et procédé pour leur préparation

(30) Priorität: 04.06.1997 DE 19723480
(43) Veröffentlichungstag der Anmeldung: 09.12.1998
(73) Patentinhaber: BASF AKTIENGESELLSCHAFT, 67056 Ludwigshafen (DE)
(72) Erfinder: Paust, Joachim, Dr., 67141 Neuhofen (DE); Kriegl, Wolfgang, 67071 Ludwigshafen (DE)

(56) Entgegenhaltungen:
- DE-A- 2 537 060
- A. RUETTIMANN ET AK: HELV. CHIM. ACTA, Bd. 63, Nr. 6, 1980, Seiten 1456-1462, XP002080806
- H. TAGUCHI ET AL: TETRAHEDRON LETT., Nr. 27, 1973, Seiten 2465-2468, XP002080807

## Beschreibung

Die Erfindung betrifft einen neuen Syntheseweg zur Herstellung von Zeaxanthin, Verfahren zur Herstellung von Zwischenprodukten dieser neuen Zeaxanthinsynthese sowie neue Zwischenprodukte dieses Verfahrens, insbesondere spezielle 7-Oxa-bicyclo[2.2.1]heptan-Derivate.

(3R,3'R)-Zeaxanthin (3,3'-Dihydroxy-β,β-carotin) der Formel I 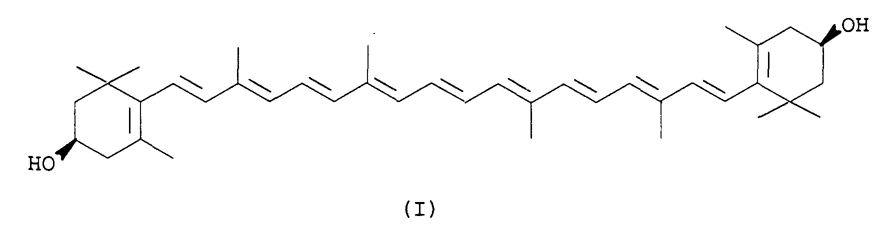 ist ein in der Natur weit verbreitetes gelbes Pigment, das u.a. im Mais, im Eidotter sowie in menschlichen und tierischen Fettgeweben vorkommt. Es wurde 1929 erstmals in kristalliner Form aus Mais isoliert und später als 3,3'-Dihydroxy-β,β-carotin identifiziert. Durch Korrelation mit Fucoxanthin konnte gezeigt werden, daß Zeaxanthin die (3R,3'R)-Konfiguration besitzt. Optisch inaktives Zeaxanthin wurde auf verschiedenen Synthesewegen schon vor längerer Zeit hergestellt (vgl. GB 812, 267 und GB 1,173,063).

Wegen des ständig steigenden Bedarfs an naturidentischen Farbstoffen hat es nicht an Versuchen gefehlt, Verfahren zur technischen Herstellung von (3R, 3'R)-Zeaxanthin zu entwickeln.

Dementsprechend wurden in der Literatur bereits verschiedene Wege zur Herstellung von Zeaxanthin beschrieben. Bewährt haben sich dabei folgende Reaktionen und Zwischenprodukte:
1) Die Verwendung von gegebenenfalls enantiomerenreinem, 4-Hydroxy-2,2,6-trimethylcyclohexanon (II) als Baustein für die cyclischen Endgruppen von Zeaxanthin und
2) die Herstellung von Zeaxanthin durch Wittig-Olefinierung von zwei Mol eines C₁₅-Triphenylphosphoniumsalzes der Formel III mit dem symmetrischen C₁₀-Dialdehyd 2,7-Dimethylocta-2,4,6-trien-1,8-dial (VIII)

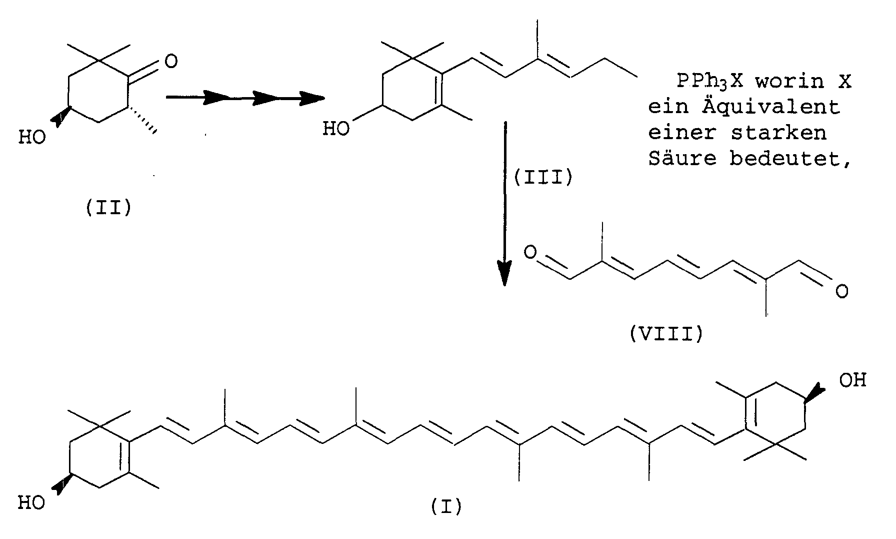

Die verschiedenen Synthesewege unterscheiden sich jedoch beträchtlich darin, auf welchem Weg man vom C₉-Baustein II zu dem benötigten C₁₅-Triphenylphosphoniumsalz gelangt.

Intensiv bearbeitet wurde das nachfolgend schematisch dargestellte Synthesekonzept C₉+C₆ → C₁₅. Beispielsweise hat man die durch Verknüpfung von 4-Hydroxy-2,2,6-trimethyl-cyclohexanon mit geschützter OH-Gruppe (2; PG = protecting group) mit dem geschützten C₆-Lithiumacetylid 3 erhältliche C₁₅-Zwischenstufe durch Behandlung mit Salzsäure (vgl. EP 100 839) oder durch Estereliminierung (vgl. EP 131 130) zu der Verbindung 4 dehydratisiert, diese in das acetylenische Phosphoniumsalz 5 übergeführt und daraus durch Partialhydrierung das C₁₅-Triphenylphosphoniumsalz der Formel III hergestellt.

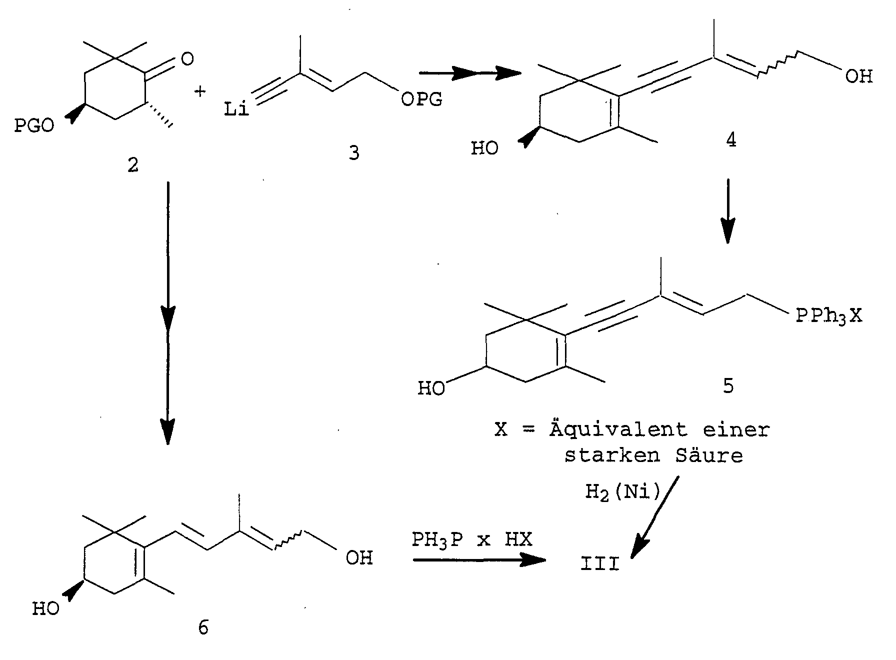

Die fermentative Herstellung von optisch aktiven [4R,6R]-4-Hydroxy-2,2,6-trimethyl-cyclohexanon ist Gegenstand von DE-A-2 537 060.

In EP 120 341 wurde beschrieben, die durch Verknüpfung von 2 und 3 erhältliche C₁₅-Zwischenstufe direkt mit Aluminiumhydriden zu reduzieren, das dabei erhaltene 3-Methyl-2,4-pentadien-1-ol-Derivat unter speziellen Bedingungen zu dehydratisieren (vgl. EP 454002) und das so erhaltene 3-Hydroxy-β-ionylidenethanol 6 in das Triphenylphosphoniumsalz der Formel III überzuführen.

Die Varianten dieses Synthesekonzepts C₉+C₆ → C₁₅ wurden in Helv. Chim. Acta 73 (1990) 861 evaluiert. Die Autoren kommen zu dem Schluß, daß eine ökonomische Herstellung von Zeaxanthin auf diesen Wegen nicht möglich ist, da die Instabilität einzelner Stufen und die Bildung von (9Z)-Isomeren zu mäßigen Gesamtausbeuten an dem Triphenylphosphoniumsalz der Formel III führen.

Stand der Technik bei der technischen Herstellung von Zeaxanathin ist das Synthesekonzept C₉+C₂+C₄ → C₁₅ (vgl. EP 283 979 und Helv. Chim. Acta 73 (1990) 868). Die Umsetzung des C₉-Hydroxyketons II zum C₁₅-Triphenylphosphoniumsalz der Formel III verläuft dabei über das C₁₁-Acetylencarbinol 7 und das C₁₅-Acetylencarbinol 8 und gelingt in einer Gesamtausbeute von 70 %.

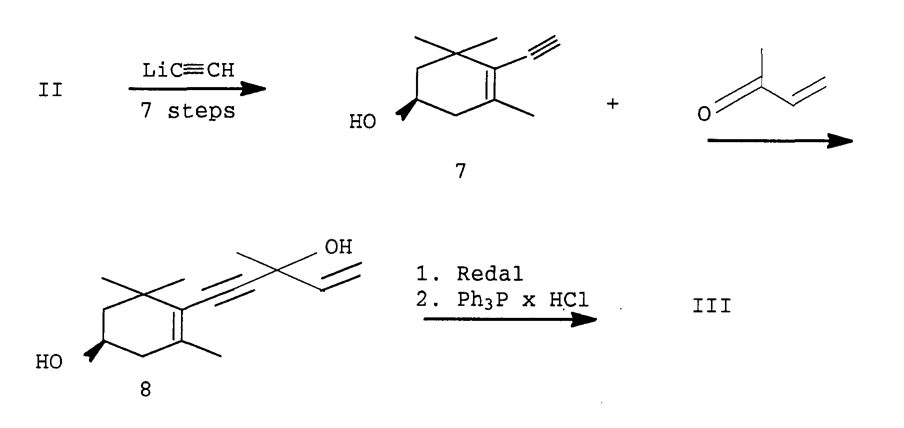

Die über 7 und 8 verlaufende Synthese des C₁₅-Triphenylphosphoniumsalzes der Formel III ist jedoch ökonomisch und ökologisch unbefriedigend. Dies rührt daher, daß die insgesamt zehn Stufen unter extrem unterschiedlichen Reaktionsbedingungen durchgeführt werden müssen. Diese überstreichen den gesamten pH-Bereich und einen Temperaturbereich von -20°C bis +130°C und erfordern abwechselnd Lösungsmittel von sehr unterschiedlicher Polarität. Die extrem unterschiedlichen Reaktionsbedingungen stellen hohe Anforderungen an die Materialien der verwendeten Apparate und haben auch zur Folge, daß dreimal Schutzgruppen eingeführt und wieder abgespalten und entsorgt werden müssen.

Es war daher die Aufgabe der Erfindung, ein neues Verfahren zur technischen Herstellung von Zeaxanthin zu entwickeln, bei dem die oben beschriebenen Nachteile des Standes der Technik nicht auftreten, d.h. ein Verfahren zu entwickeln, mit dessen Hilfe man ausgehend von dem gut zugänglichen optisch aktiven (4R)-Hydroxy-2,2,6-trimethyl-cyclohexanon der Formel II in weniger und technisch leichter realisierbaren Reaktionsstufen in guten Ausbeuten zu dem gewünschten Zeaxanthin gelangt.

Es war weiterhin Aufgabe der Erfindung, neue Zwischenprodukte für das neue Zeaxanthin-Verfahren zur Verfügung zu stellen und vorteilhafte Verfahren zu deren Herstellung zu entwickeln.

Es wurde nun überraschenderweise gefunden, daß man Zeaxanthin ausgehend von dem gut zugänglichen (4R)-4-Hydroxy-2,2,6-trimethyl-cyclohexanon der Formel II in nur 5 technisch relativ leicht durchführbaren Reaktionsschritten über interessante neue Zwischenprodukte und die isomeren, chemisch gleichwertigen C₁₅-Triphenylphosphoniumsalze der Formel III und iso-III 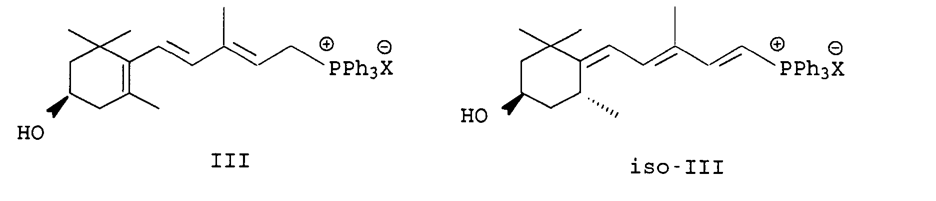 in guten Ausbeuten herstellen kann, wobei sich die Einführung und Wiederabspaltung von Schutzgruppen erübrigt.

Gegenstand der Erfindung ist daher ein Verfahren zur Herstellung von (3R, 3'R)-Zeaxanthin der Formel I 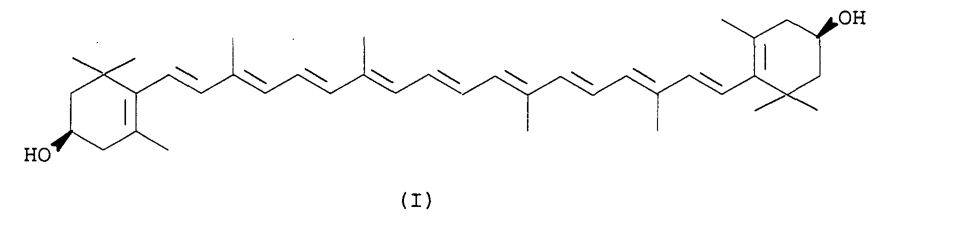 nach dem neuen Syntheseschema C₉ + C₁ + C₃ + C₂ → C₁₅; 2 C₁₅ + C₁₀ → C₄₀, das dadurch gekennzeichnet ist, daß man
A. (4R)-4-Hydroxy-2,2,6-trimethyl-cyclohexanon der Formel II 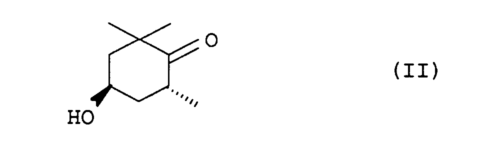 bei Temperaturen von -120 bis -40°C in einem inerten Lösungsmittel mit Dichlormethyl-Lithium umsetzt,
B. das beim Erwärmen des Reaktionsgemisches auf 20 bis 60°C erhaltene neue (4R,6R)-1-Formyl-2,2,6-trimethyl-7-oxa-bicyclo[2.2.1]-heptan der Formel IV 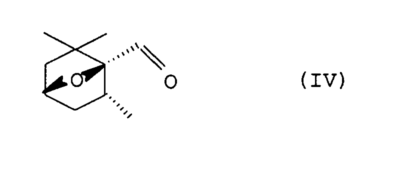 entweder in isolierter Form oder aber direkt in Form des erhaltenen Reaktionsgemisches mit Aceton oder einem (2-Oxopropyl)-phosphonsäuredialkylester umsetzt,
C. das dabei erhaltene neue (4R,6R)-1-(3-Oxo-but-l-en-1-yl)-2,2,6-trimethyl-7-oxa-bicyclo[2.2.1]heptan der Formel V 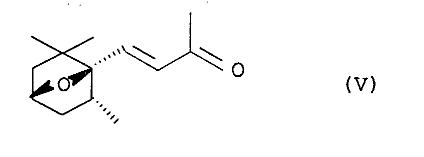 in an sich bekannter Weise durch Vinylierung oder Ethinylierung und anschließende Partialhydrierung in das neue (4R,6R)-1-(3-Hydroxy-3-methyl-penta-1,4-dien-1-yl)-2,2,6-trimethyl-7-oxa-bicyclo[2.2.1]-heptan der Formel VI überführt 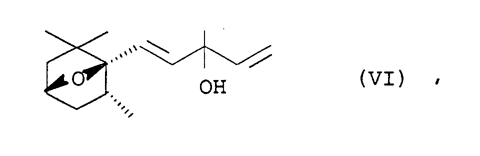
D. dieses mit einem Triarylphosphin, vorzugsweise Triphenylphosphin, und einer starken Säure zu einem Gemisch aus dem C₁₅-Triarylphosphoniumsalz der allgemeinen Formel III und dem neuen isomeren der allgemeinen Formel iso-III 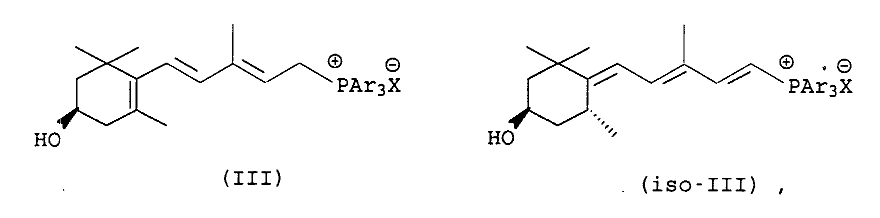 in der Ar für einen Arylrest, vorzugsweise einen ggf. substituierten Phenylrest steht und
   X für ein Äquivalent eines Anions einer starken Säure, insbesondere für Cl, Br oder (HSO₄) steht, umsetzt,
und E. das erhaltene Gemisch aus den C₁₅-Triarylphosphoniumsalzen III und dem neuen iso-III durch eine doppelte Wittig-Olefinierungsreaktion mit 2,7-Dimethylocta-2,4,6-trien-1,8-dial der Formel VIII 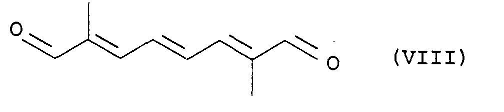 in Zeaxanthin der Formel I überführt.

Gegenstand der Erfindung ist weiterhin das neue Zwischenprodukt (4R,6R)-1-Formyl-2,2,6-trimethyl-7-oxa-bicyclo[2.2.1]heptan der Formel IV 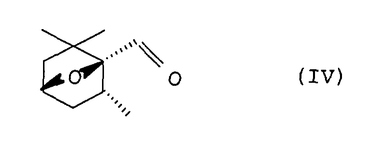 sowie ein Verfahren zu dessen Herstellung, das dadurch gekennzeichnet ist, daß man (4R)-4-Hydroxy-2,2,6-trimethyl-cylohexanon der Formel II 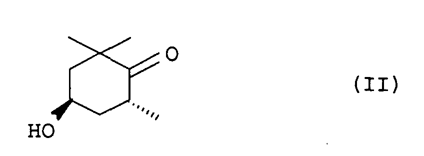 mit dem durch Metallieren von Dichlormethan mit Alkyl-Lithiumverbindungen bei Temperaturen von -120 bis -70°C oder mit Lithium-Dialkylaminen bei Temperaturen von -70 bis -40°C erhaltenen Dichlormethyl-Lithium bei Temperaturen von -120 bis -40°C in einem inerten Lösungsmittel umsetzt und das erhaltene Reaktionsgemisch, ggf. in Gegenwart von einem Alkalialkoholat oder einem Alkalihydroxid, auf Temperaturen von 20 bis 60°C erwärmt.

Gegenstand der Erfindung ist außerdem das neue Zwischenprodukt (4,R,6R)-1-(3-Oxo-but-1-en-1-yl)-2,2,6-trimethyl-7-oxa-bicyclo[2.2.1]heptan der Formel V 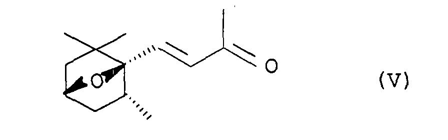 sowie ein Verfahren zu dessen Herstellung, das dadurch gekennzeichnet ist, daß man das neue (4R,6R)-1-Formyl-2,2,6-trimethyl-7-oxa-bicyclo[2.2.1]heptan der Formel IV 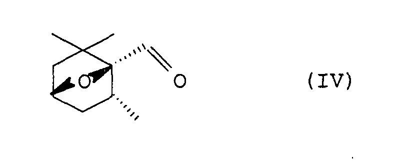 entweder in isolierter Form oder direkt in Form des bei der Umsetzung von (4R,6R)-4-Hydroxy-2,2,6-trimethyl-cyclohexan der Formel II 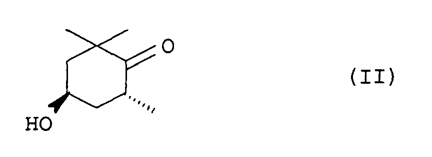 mit dem durch Metallieren von Dichlormethan mit Alkyl-Lithiumverbindungen bei Temperaturen von -120 bis -70°C oder mit Lithium-Dialkylaminen bei Temperaturen von -70 bis -40°C erhaltenen Dichlormethyl-Lithium bei -120 bis -40°C in einem inerten Lösungsmittel und anschließendes Erwärmen auf 20 bis 60°C erhaltene Reaktionsgemisch in einer Aldolkondensation mit Aceton umsetzt.

Gegenstand der Erfindung ist darüber hinaus das neue Zwischenprodukt (4R,6R)-1-(3-Hydroxy-3-methyl-penta-1,4-dien-1-yl)-2,2,6-trimethyl-7-oxa-bicyclo[2.2.1]heptan der Formel VI 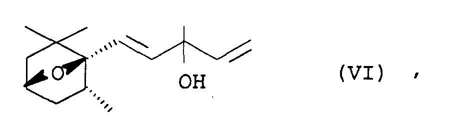 sowie ein Verfahren zu dessen Herstellung, das dadurch gekennzeichnet ist, daß man das neue (4R,6R)-1-(3-Oxo-but-1-en-1-yl)-2,2,6-trimethyl-7-oxa-bicyclo[2.2.1]heptan der Formel V 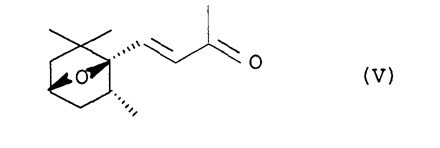 in an sich bekannter Weise vinyliert oder zunächst ethinyliert und anschließend partiell hydriert.

Gegenstand der Erfindung ist auch ein Verfahren zur Herstellung eines Gemisches aus dem C₁₅-Triarylphosphoniumsalz der allgemeinen Formel III und dem neuen isomeren der Formel iso-III 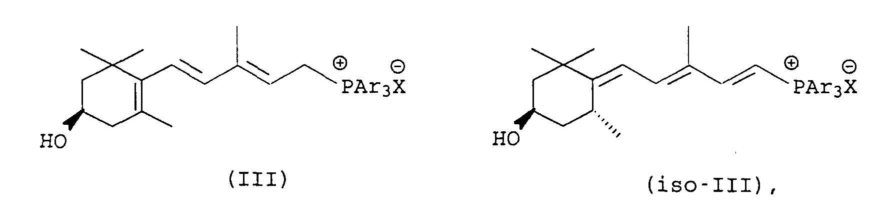 in der Ar für einen Arylrest, vorzugsweise einen ggf. substituierten Phenylrest steht und
X für ein Äquivalent eines Anions einer starken Säure, insbesondere für Cl, Br oder (HSO₄) steht, das dadurch gekennzeichnet ist, daß man das neue (4R,6R)-1-(3-Hydroxy-3-methylpenta-1,4-dien-1-yl)-2,2,6-trimethyl-7-oxa-bicyclo[2.2.1]heptan der Formel VI 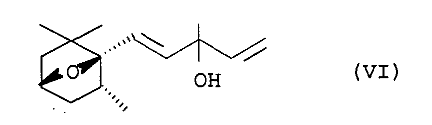 mit einem Triarylphosphin und einer starken Säure umsetzt, sowie als neue Zwischenprodukte [5-(4R,6R)-(4-Hydroxy-2,2,6-trimethylcyclohex-1-yliden)-3-methyl-penta-1,3-dien]-triphenylphosphoniumsalze der allgemeinen Formel iso-III 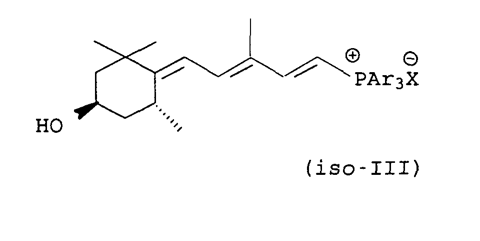 im Gemisch mit den entsprechenden [5-(4R)-(4-Hydroxy-2,2,6-trimethyl-cyclohex-1-en-1-yl)-3-methyl-penta-2,4-dienyl]-triarylphosphoniumsalzen der allgemeinen Formel III 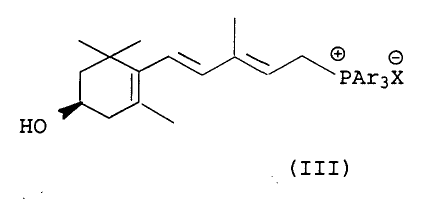 in denen Ar für einen Arylrest, vorzugsweise einen gegebenenfalls substituierten Phenylrest steht und X für ein Äquivalent des Anions einer starken Säure steht.

### Zu Reaktionsschritt A:

Das als Ausgangsverbindung verwendete optisch aktive (4R)-4-Hydroxy-2,2,6-trimethyl-cyclohexanon der Formel II ist eine bekannte Verbindung, die technisch recht gut herstellbar ist (vgl. Helv. Chim. Acta 73 (1990), 861).

Die Reaktion von Ketonen mit Dichlormethyl-Lithium ist literaturbekannt (vgl. Tetrahedron Letters 27 (1973), 2465) und führt im allgemeinen zu Lithiumdichlormethylcarbinolaten, die beim Erwärmen unter Eliminierung von Lithiumchlorid zu α-Chlor-aldehyden umlagern.

Im Falle des (4R)-4-Hydroxy-2,2,6-trimethyl-cyclohexanons der Formel II führt die Umsetzung mit Dichlormethyl-Lithium überraschenderweise zu dem neuen C₁₀-Aldehyd (4R,6R)-1-Formyl-2,2,6-trimethyl-7-oxa-bicyclo[2.2.1]heptan der Formel IV.

Das für diesen Reaktionsschritt benötigte Dichlormethyl-Lithium wird unter den üblichen Bedingungen hergestellt, und zwar durch Metallierung von Dichlormethan mit Alkyl-Lithiumverbindungen, insbesondere mit dem handelsüblichen n-Butyl-Lithium, bei Temperaturen von -120 bis -70°C, vorzugsweise -90 bis -60°C, oder aber mit einem Lithium-dialkylamin, wie Lithium-diisopropylamin und Lithium-dicyclohexylamin, insbesondere dem handelsüblichen Lithium-diisopropylamin bei Temperaturen von -70 bis -40°C, vorzugsweise -70 bis -30°C. Die Umsetzung des Cyclohexanons der Formel II zu dem bicyclischen Aldehyd der Formel IV erfolgt im allgemeinen durch langsame Zugabe dieses Ketons bei den oben angegebenen Temperaturen zu der hergestellten Lösung von Dichlormethyl-Lithium und anschließendes langsames Erwärmen auf Temperaturen von 20 bis 60°C. Das Erwärmen des Reaktionsansatzes erfolgt mit Vorteil in Gegenwart von katalytischen Mengen eines Alkalialkoholats, wie Kalium-tert.-butylat oder Natriummethylatpulver oder eines Alkalihydroxids, wie LiOH, NaOH, KOH, vorzugsweise dem billigen NaOH. Die Aufarbeitung des Reaktionsansatzes erfolgt im allgemeinen durch Versetzen mit Wasser und Extraktion mit einem mit Wasser nicht oder nur schlecht mischbaren Lösungsmittel, wie geeigneten Kohlenwasserstoffen, insbesondere Cyclohexan und Hexan oder mit einem Ether, wie Diethylether oder Methyl - tert. - butylether.

Die Ausbeute für diese Umsetzung beträgt etwa 85 % der Theorie.

Will man aus dem bicyclischen Aldehyd der Formel IV Zeaxanthin herstellen, kann ohne Ausbeuteverschlechterung auf die Isolierung des Aldehyds verzichtet werden, d.h. der Aldehyd kann direkt in Form des bei diesem Reaktionsschritt erhaltenen Reaktionsgemisches weiter umgesetzt werden.

### Zu Reaktionsschritt B:

Die Umsetzung des neuen bicyclischen C₁₀-Aldehyds der Formel IV zu dem neuen bicyclischen C₁₃-Keton der Formel V mit Aceton kann prinzipiell nach bekannten Vorschriften erfolgen, beispielsweise durch eine basenkatalysierte Kondensation mit Aceton. Mit Vorteil verwendet man bei dieser Kondensation das Aceton im Überschuß als Lösungsmittel. Als basische Katalysatoren eignen sich beispielsweise Alkalimetallhydroxide und Erdalkalimetallhydroxide, insbesondere Alkalimetallhydoxide. In einer bevorzugten Ausführungsform dieser Kondensation mit Aceton wird der bicyclische Aldehyd der Formel IV oder das diesen Aldehyd enthaltende Reaktionsgemisch mit etwa der 10- bis 20-fachen molaren Menge an Aceton versetzt und die Lösung nach Zugabe einer etwa äquimolaren Menge NaOH-Pulver ca. 1 Stunde unter Rückfluß zum Sieden erhitzt.

Eine weitere an sich bekannte Möglichkeit den C₁₀-Aldehyd der Formel IV in das C₁₃-Keton der Formel V zu überführen besteht darin, daß man den C₁₀-Aldehyd mit (2-Oxo-propyl)-phosphonsäurediethylester umsetzt. Als Basen eignen sich in diesem Fall insbesondere Alkalialkoholate niederer Alkanole, wie Natriummethylat. Die Aufarbeitung des Reaktionsgemisches erfolgt im allgemeinen auf übliche Weise, beispielsweise dadurch, daß man Feststoffe im Reaktionsgemisch durch Filtration abtrennt, das Filtrat weitgehend eindampft, den Rückstand in einem unpolaren organischen Lösungsmittel aufnimmt, diese Lösung mit Wasser wäscht, die organische Phase einengt und das neue Keton der Formel V destillativ reinigt.

### Zu Schritt C:

Auch die Überführung des neuen C₁₃-Ketons der Formel V in das neue C₁₅-Carbinol der Formel VI kann nach prinzipiell bekannten Vorschriften erfolgen. Beispielsweise kann man das Keton zunächst durch 1,2-Addition von Lithium- oder Natriumacetylid in das neue (4R,6R)-1-(3-Hydroxy-3-methyl-penta-1-en-4-in-1-yl)-2,2,6-trimethyl-7-oxa-bicyclo[2.2.1]heptan überführen und dieses anschließend partiell zu dem neuen C₁₅-Carbinol der Formel VI hydrieren. Die Partialhydrierung kann dann unter den als Lindlar-Hydrierung bekannten Bedingungen durchgeführt werden. Bezüglich näherer Einzelheiten zur Durchführung der Ethinylierung von Ketonen und der Partialhydrierung der erhaltenen Alkine verweisen wir beispielsweise auf W. Oroshnik et al., J. Am. Chem. Soc. 1952, 74 und 295 bzw. H. Lindlar,Helv.Chim. Acta 35 (1952), 35 und 445.

Man kann aber auch das neue C₁₃-Keton der Formel V in nur einem Schritt durch 1,2-Addition von Vinyl-Lithium oder Vinylmagnesiumchlorid direkt in das neue C₁₅-Carbinol der Formel VI überführen. Bevorzugte Lösungsmittel für beide Umsetzungen mit metallorganischen Verbindungen sind flüssiger Ammoniak und Tetrahydrofuran. Bezüglich näherer Einzelheiten über die Durchführung von Vinylierungen von Ketonen verweisen wir beispielsweise auf Y. Ishikawa et al., Bull.Chem. Soc. Japan 37 (1964), 207.

Die Aufarbeitung des Reaktionsgemisches erfolgt im allgemeinen auf übliche Weise. Beispielsweise kann man das Reaktionsgemisch eindampfen, den Rückstand mit Wasser und einem geeigneten unpolaren organischen Lösungsmittel behandeln, die abgetrennte organische Phase einengen und das so erhaltene Rohprodukt erforderlichenfalls destillativ reinigen.

### Zu Reaktionsschritt D:

Auch die Umsetzung des neuen C₁₅-Carbinols der Formel VI zu dem Gemisch aus dem C₁₅-Triarylphosponiumsalz der allgemeinen Formel III und dem neuen isomeren C₁₅-Triarylphosphiniumsalz der Formel iso-III erfolgt nach bekannten Vorschriften. Beispielsweise kann man das C₁₅-Carbinol bei Temperaturen von -10 bis +40°C in einem organischen Lösungsmittel mit den entsprechenden Triarylphosphoniumsalzen umsetzen. Als Lösungsmittel verwendet man dabei mit Vorteil aprotische Lösungsmittel, wie Dichlormethan oder Dimethylformamid. Bezüglich näherer Einzelheiten der Reaktion überweisen wir auf M. Rosenberger et al., J. Org. Chem. 47 (1982), 47 und 2130. Als Triarylphospinsalze verwendet man vorzugsweise Triphenylphosphinhydrohalogenide oder Triphenylphosphinhydrogensulfat, insbesondere Triphenylphosphinhydrobromid oder -hydrochlorid.

Die C₁₅-Triarylphosphoniumsalze III und iso-III bilden sich je nach Konfiguration des C₁₅-Carbinols der Formel VI sowie in Abhängigkeit von den Reaktionsbedingungen in unterschiedlichem MolVerhältnis. Sie können entweder direkt in Form des erhaltenen Reaktionsgemisches weiter umgesetzt oder aber isoliert werden. Zur Isolierung destilliert man beispielsweise das Lösungemittel unter gleichzeitiger Zugabe von Acetessigsäureethylester oder von Toluol ab und saugt das in kristalliner Form anfallende Gemisch aus den Triarylphosphoniumsalzen III und iso-III ab.

### Zu Reaktionsschritt E:

Die Umsetzung der C₁₅-Triarylphosphoniumsalze der Formel III mit dem C₁₀-Dialdehyd 2,7-Dimethyl-octa-2,4,6-trien-1,8-dial der Formel VIII ist aus der Literatur bekannt (vgl. beispielsweise Helv. Chim. Acta, Vol 63 (1980), 1456-1462, besonders 1460-61 und loc.cit. Vol. 73 (1990), 864). Sie gelingt besonders gut in aprotischen Lösungsmitteln, wie Dichlormethan, mit Lithium-organischen Verbindungen, wie sie in Reaktionsstufe A genannt wurden, oder aber mit 1,2-Epoxy-butan in Ethanol unter Erhitzen unter Rückfluß.

Die neuen isomeren C₁₅-Triarylphosphiniumsalze der Formel iso-III reagieren bei dieser Umsetzung unter Bildung des identischen Produkts: (3R, 3'R)-Zeaxanthin.

Mit Hilfe des erfindungsgemäßen Verfahrens ist es möglich, das als naturidentischer Farbstoff begehrte Zeaxanthin aus dem gut zugänglichen (4R)-4-Hydroxy-2,2,6-trimethyl-cyclohexanon der Formel II unter weitgehender Vermeidung der Nachteile des Standes der Technik in nur 5 relativ gut auch technisch durchführbaren Stufen über interssante neue Zwischenprodukte in guten Ausbeuten herzustellen.

### Experimenteller Teil

### Beispiel 1:

### (4R,6R)-1-Formyl-2,2,6-trimethyl-7-oxa-bicyclo[2.2.1]heptan (IV)

- a) Umsetzung:: Eine Lösung von 45 ml (0,54 mol) Dichlormethan in 250 ml eines Gemisches aus Tetrahydrofuran (THF) und Diethylether (4/1) wurde in ca. 30 Minuten (min) bei -70°C mit 284 ml einer Lösung von n-Butyllithium (0,45 mol) in Hexan versetzt und weitere 30 min bei -70°C gerührt. Zu dieser Lösung gab man tropfenweise bei -70°C 22,8 g (0,15 mol) (4R)-4-Hydroxy-2,2,6-trimethyl-cyclohexanon in 40 ml eines Gemisches aus THF und Diethylether (4/1) und ließ den Ansatz dann in ca. 2 Stunden (h) auf Raumtemperatur (RT) kommen. Danach setzte man 2,7 g (0,05 mol) Natriummethylatpulver zu und erwärmte den Ansatz langsam auf 50°C bis die Gasentwicklung beendet war.
- b) Aufarbeitung:: Der gemäß Beispiel la erhaltene Reaktionsansatz wurden bei RT mit 200 ml Wasser versetzt, die Oberphase abgetrennt und die Wasserphase dreimal mit je 150 ml Hexan extrahiert. Die organischen Phasen wurden vereinigt, zweimal mit 150 ml Wasser gewaschen und eingedampft (Rotationsverdampfer). Der Rückstand von 25,5 g wurde über eine Brücke destilliert. Man erhielt 22,1 g 1-Formyl-2,2,6-trimethyl-7-oxa-bicyclo[2.2.1]heptan mit einem Kp = 54-61°C bei 1,5 mbar, das nach einer gaschromatographischen Gehaltsbestimmung (GC) 90%ig ist (entsprechend einer Ausbeute von 85 % der Theorie). Als Nebenprodukt wurde 1-Hydroxymethyl-2,2,6-trimethyl-7-oxa-bicyclo[2.2.1]heptan (ca. 4 % d.Th.) bestimmt. Die Struktur des Verfahrensproduktes ergab sich beispielsweise aus dem 'H-NMR-Spektrum: 250 MHz, (C₆D₆), δ=1,09 (d, J = 8 Hz, 3 H), 4,16 (t, J = 6 Hz, 1H), 9,94 (s, 1 H) ; [α]_{D}²⁵ = -20,7 (c=1, Ethanol).

### Beispiel 2

### Herstellung von (4R,6R)-1-(Oxobut-1-en-1-yl)-2,2,6-trimethyl-7-oxa-bicyclo[2.2.1]-heptan (V)

- a) Umsetzung:: Eine Lösung von 45 ml Dichlormethan in 250 ml eines Gemisches aus THF und Diethylether (4/1) wurde in ca. 30 min bei -70°C mit 284 ml einer Lösung von n-Butyllithium (0,45 mol) in Hexan versetzt und weitere 30 min bei -70°C gerührt. Zu dieser Lösung gab man tropfenweise bei 70°C 22,8 g (0,15 mol) 4R-Hydroxy-2,2,6-trimethyl-cyclohexanon in 40 ml THF/Diethylether (4/1) und ließ den Ansatz dann innerhalb von 2 h auf RT kommen. Danach wurde mit 2,7 g Natriummethylatpulver versetzt und langsam auf 50°C erwärmt bis die Gasentwicklung beendet war. Danach wurde das Reaktionsgemisch mit 250 ml Aceton und 8,5 g (0,2 mol) NaOH-Pulver versetzt und dann 20 min unter Rückfluß zum Sieden (ca. 57°C) erhitzt.
- b) Aufarbeitung:: Man goß den Ansatz in 600 ml Wasser, trennte die organische Phase ab und wusch zweimal mit je 150 ml Wasser. Die vereinigten Wasserphasen wurden zweimal mit je 150 ml Hexan extrahiert. Die organischen Phasen wurden vereinigt und am Rotationsverdampfer eingeengt. Man erhielt 37,2 g Rückstand. Vakuumdestillation dieses Rückstandes in einer 10 cm Einstichkolonne lieferte 27,9 g 1-(Oxobut-1-en-yl)-2,2,6-trimethyl-7-oxa-bicyclo[2.2.1]heptan mit einem Kp=90-96°C bei 1 mbar, welches nach GC ca. 95 %ig war (entsprechend einer Ausbeute von 85 % d.Th.). Die Struktur des neuen Produktes ergab sich durch 'H-NMR- und IR-spektroskopische Untersuchung; 250 MHz (C₆D₆); δ=1,83 (s, 3H, CO-CH₃), 4,20 (t, 1H, OCH), 6.50 und 7.00 (d,d J = 20 Hz, CH=CH); γ=1672 cm⁻¹ (C=O), 1630 cm⁻¹ (CH=CH); [α]_{D}²⁵ = -20,7 (c=1, Ethanol).

### Beispiel 3

### Herstellung von (4R,6R)-1-(3-Hydroxy-3-methylpenta-1,4-dien-1-yl)-2,2,6-trimethyl-7-oxa-bicyclo[2.2.1]-heptan (VI)

- a) Umsetzung:: Zu 80 ml einer 1,3 molaren Lösung von Vinylmagnesiumchlorid (ca. 100 mmol) in THF gab man in 30 min tropfenweise unter Kühlung mit einem Eisbad eine Lösung von 15,6 g (70 mmol) des gemäß Beispiel 2 hergestellten, destillierten Ketons in 20 ml THF. Die Reaktionstemperatur wurde dabei auf 18-22°C gehalten.
- b) Aufarbeitung: Der gemäß a) erhaltene Reaktionsansatz wurde noch 30 min gerührt und dann unter Kühlung mit einem Eis/Kochsalz-Bad mit 180 ml einer 25 %igen wäßrigen Natronlauge und 200 ml Methyl-t-butylether (MTB) versetzt. Man trennte die organische Phase ab, wusch die Wasserphase zweimal mit je 100 ml MTB und dampfte die vereinigten MTB-Phasen am Rotationsverdampfer ein. Man erhielt 22 g 1-(3-Hydroxy-3-methyl-penta-1,4-dien-1-yl)-2,2,6-trimethyl-7-oxa-bicyclo[2.2.1]heptan (GC-Gehaltsbestimmung: 90 Fl.%.), Vakuumdestillation des erhaltenen Produktes in einer 10 cm Einstichkolonne lieferte bei Kp. 94 bis 99°C bei 1 mbar 18,9 g des obengenannten Produktes, das nach GC 92 %ig war, entsprechend einer Ausbeute von 95% d.Th. Die Strukturzuordnung der neuen Verbindung erfolgte 'H-NMR-spektroskopisch: 250 MHz (DMSO), δ=0,75; 0,95, 1.11 (3s, 3CH₃), 2,25 (m-H-C₆), 4.26 (t,H-C₄), 4,72 (s,OH), 4,88-5,94 (5-Vinyl-H). [α]_{D}²⁵ = -27,5 (c=0,5, Methanol)

### Beispiel 4

### Herstellung von [5-(4R)-(4-Hydroxy-2,2,6-trimethyl-cyclohex-1-en-yl)-3-methyl-penta-2,4-dienyl]-triphenylphosphiniumbromid (IIIa) und [5-(4R,6R)-(4-Hydroxy-2,2,6-trimethyl-cyclohex-1-yliden)-3-methyl-penta-1,3-dienyl]-triphenylphosphiniumbromid (iso-IIIa)

Zu einer Lösung von 3,43 g (10 mmol) Triphenylphosphinhydrobromid und 0,13 g (0,5 mmol) Triphenylphosphin in 30 ml Dichlormethan wurde unter Eisbadkühlung tropfenweise eine Lösung von 2,47 g (10 mmol) eines gemäß Beispiel 3 hergestellten (4R, 6R)-1-(3-Hydroxy-3-methyl-penta-1,4-dien-1-yl)-2,2,6-trimethyl-7-oxabicyclo[2.2.1]heptans (Gehalt 95 %) in 5 ml Dichlormethan addiert. Anschließend wurde unter Erwärmung auf RT 1 h weitergerührt.

Die erhaltene Reaktionslösung wurde anschließend innerhalb von 1 h tropfenweise in 600 ml Diethylether eingetragen. Nach 2 h wurden die gebildeten Kristalle abgesaugt, mit 50 ml Diethylether gewaschen und im Stickstoffstrom getrocknet. 5,6 g, entsprechend 100 % der Theorie, eines Gemisches der obengenannten isomeren C₁₅-Triphenylphosphoniumbromide etwa zu gleichen Teilen wurden erhalten. Die Strukturordnung wurde aufgrund von 'H-NMR-spetroskopischen Daten vorgenommen:
250 MHz, (DCCl₃):
Verbindung IIIa δ =1,33 (d, CH₃), 1,60 (s, CH₃), 386-4,02 (m, HCO), 4,80-5,00 (m, CH₂-P), 5,24-5,39 (m, H-C₂) 5,90 (s, H-C₄ und H-C₅);
Verbindung iso-IIIa δ = 2,19 (s, CH₃), 3,19-3,26 (m, HCO), 6,32 und 6,56 (2d, J=20 Hz, H-C_{4,5}), 6,78 und 6,88 (2d, J=10 Hz, H-C_{2,3}).

### Beispiel 5

### Herstellung von (3R, 3'R)-Zeaxanthin (I)

Unter Ausschluß von Feuchtigkeit und Luftsauerstoff wurden folgende Umsetzung und Aufarbeitung vorgenommen.
- a) Umsetzung:: Eine Lösung von 1,2 g (12 mmol) Diisopropylamin in 50 ml Diethylether wurde bei -10°C tropfenweise mit 7,5 ml einer 1,6-molaren Lösung (insges. 12 mmol) von n-Butyllithium in Hexan versetzt. Anschließend wurde noch 1h bei -10°C nachgerührt. 5,6 g (10 mmol) eines gemäß Beispiel 4 erhaltenen 1:1-Gemisches der Triphenylphosphoniumbromide III und iso-III wurden bei -10°C in 50 ml Diethylether suspendiert. Zu dieser Suspension wurde bei -10°C tropfenweise die - wie oben beschrieben - erhaltene Lösung von Lithiumdiisopropylamin in Diethylether eingetragen und noch 1 h bei -10°C nachgerührt. In die so erhaltene tiefrote Lösung des C₁₅-Ylids wurde tropfenweise bei -10°C eine Lösung von 0,57 g (3,5 mmol) 2,6-Dimethyl-octa-2,4,6-trien-1,8-dialdehyd (VIII) in 20 ml Dichlormethan eingetragen und das Reaktionsgemisch noch 2 h unter Rückfluß zum Sieden erhitzt.
- b) Aufarbeitung:: Das in Beispiel 5a erhaltene Reaktionsgemisch wurde auf -10°C gekühlt und tropfenweise mit 70 ml Methanol versetzt. Anschließend wurden die ausgeschiedenen Kristalle abfiltriert, der Filterkuchen mit 30 ml Methanol gewaschen und im Stickstoffstrom getrocknet. Man erhielt 1,8 g (3R, 3'R)-Zeaxanthin, entsprechend einer Ausbeute von 90% der Theorie, bezogen auf eingesetzten Dialdehyd VII. Nach HPLC-Analyse überwiegt in dem erhaltenen Zeaxanthin das all-E-Isomere.
Das erhaltene Zeaxanthin wurde durch thermische Isomerisierung und Kristallisation aus Dichlormethan/Ethanol gereinigt und in (all-E)-Zeaxanthin überführt. Die Ausbeute an reinem (all-E)-Zeaxanthin betrug 1,6 g, entsprechend 81 % der Theorie, bezogen auf eingesetzten Dialdehyd VIII.

## Patentansprüche

1. Verfahren zur Herstellung von (3R, 3'R)-Zeaxanthin der allgemeinen Formel I 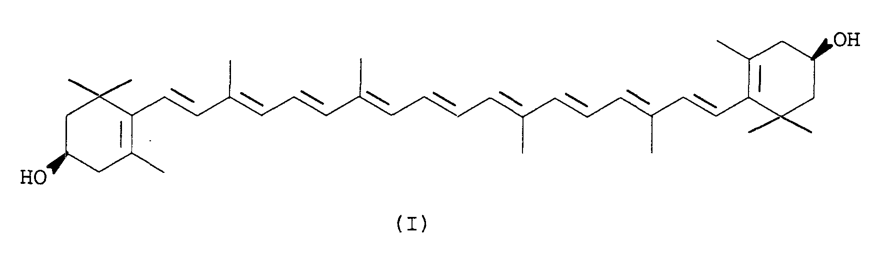 dadurch gekennzeichnet, daß man
A. (4R)-4-Hydroxy-2,2,6-trimethyl-cyclohexanon der Formel II 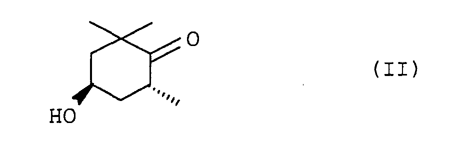 bei Temperaturen von -120 bis -40°C in einem inerten Lösungsmittel mit Dichlormethyl-Lithium umsetzt,
B. das beim Erwärmen des Reaktionsgemisches auf 20 bis 60°C erhaltene neue (4R,6R)-1-Formyl-2,2,6-trimethyl-7-oxa-bicyclo[2.2.1]-heptan der Formel IV 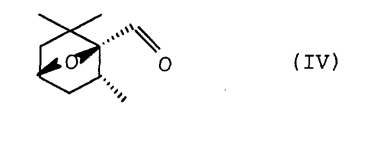 entweder in isolierter Form oder aber direkt in Form des erhaltenen Reaktionsgemisches mit Aceton oder einem (2-Oxo-propyl)-phosphonsäuredialkylester umsetzt,
C. das dabei erhaltene neue (4R,6R)-1-(3-Oxo--but-1-en-1-yl)-2,2,6-trimethyl-7-oxo-bicyclo[2.2.1]heptan der Formel V 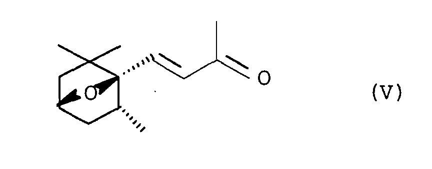 in an sich bekannter Weise durch Vinylierung oder Ethinylierung und anschließende Partialhydrierung in das neue (4R,6R)-1-(3-Hydroxy-3-methylpenta-1,4-dien-1-yl)-2,2,6-trimethyl-7-oxa-bicyclo[2.2.1]-heptan der Formel VI überführt 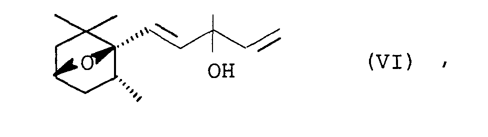
D. dieses mit einem Triarylphosphin, vorzugsweise Triphenylphosphin und einer starken Säure zu einem Gemisch aus dem C₁₅-Triarylphosphoniumsalz der allgemeinen Formel III und dem neuen isomeren der allgemeinen Formel iso-III 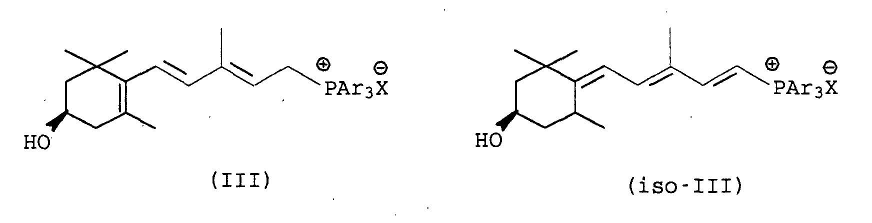 in der Ar für einen Arylrest, vorzugsweise einen ggf. substituierten Phenylrest steht und
X für ein Äquivalent eines Anions einer starken Säure, insbesondere für Cl, Br oder (HSO₄) steht, umsetzt,
und
E. jeweils etwa 2 Mol des erhaltenen Gemisches aus den C₁₅-Triarylphosphoniumsalzen III und dem neuen iso-III durch eine doppelte Wittig-Olefinierungsreaktion mit 2,7-Dimethyl-octa-2,4,6-trien-1,8-dial der Formel VIII 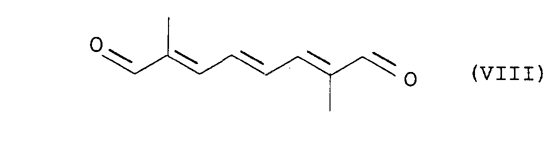 in Zeaxanthin der Formel I überführt.

2. Verfahren zur Herstellung von dem neuen (4R,6R)-1-Formyl-2,2,6-trimethyl-7-oxa-bicyclo[2.2.1]heptan der Formel IV 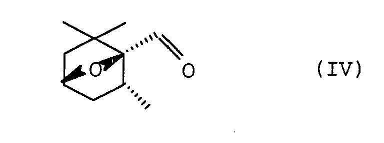 dadurch gekennzeichnet, daß man (4R)-Hydroxy-2,2,6-trimethylcylohexanon der Formel II 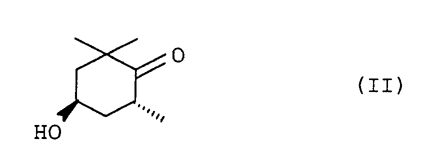 bei Temperaturen von -120 bis -40°C in einem inerten Lösungsmittel mit Dichlormethyl-Lithium umsetzt und das erhaltene Reaktionsgemisch ggf. in Gegenwart von einem Alkalialkoholat oder einem Alkalihydroxid auf Temperaturen von 20 bis 60°C erwärmt.

3. (4R,6R)-1-Formyl-2,2,6-trimethyl-7-oxa-bicyclo[2.2.1]heptan der Formel IV

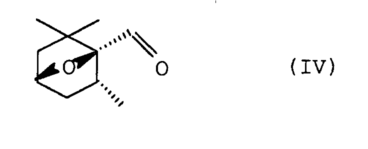

4. Verfahren zur Herstellung von dem neuen (4R,6R)-1-(3-Oxobut-1-en-1-yl)-2,2,6-trimethyl-7-oxa-bicyclo[2.2.1]heptan der Formel V 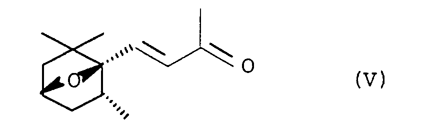 dadurch gekennzeichnet, daß man das neue (4R,6R)-1-Formyl-2,2,6-trimethyl-7-oxa-bicyclo[2.2.1]-heptan der Formel IV 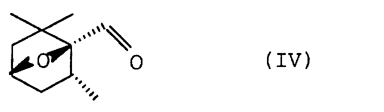 entweder in isolierter Form oder direkt in Form des bei der Umsetzung von (4R)-4-Hydroxy-2,2,6-trimethyl-cyclohexanon der Formel II 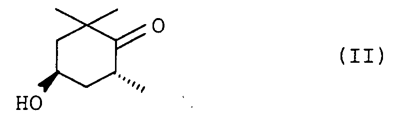 mit dem durch Metallieren von Dichlormethan mit Alkyl-Lithiumverbindungen bei Temperaturen von -120 bis -70°C oder mit Lithium-Dialkylaminen bei Temperaturen von -70°C bis -40°C erhaltenen Dichlormethyl-Lithium bei Temperaturen von -120 bis -40°C in einem inerten Lösungsmittel und anschließendes Erwärmen auf 20 bis 60°C erhaltene Reaktionsgemisch in einer Aldolkondensation mit Aceton umsetzt.

5. (4R,6R)-1-(3-Oxo-but-1-en-1-yl)-2,2,6-trimethyl-7-oxa-bicyclo[2.2.1]heptan der Formel V

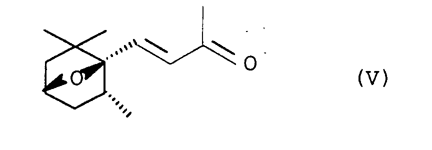

6. Verfahren zur Herstellung des neuen (4R,6R)-1-(3-Hydroxy-3-methyl-penta-1,4-dien-1-yl)-2,2,6-trimethyl-7-oxa-bicyclo[2.2.1]-heptan der Formel VI 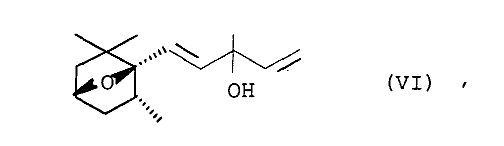 dadurch gekennzeichnet, daß man das neue (4R,6R)-1-(3-Oxobut-1-en-1-yl)-2,2,6-trimethyl-7-oxa-bicyclo[2.2.1]heptan der Formel V 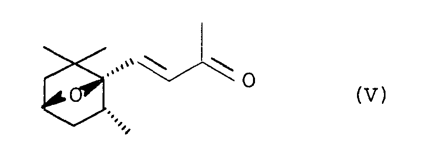 in an sich bekannter Weise vinyliert oder zunächst ethinyliert und anschließend partiell hydriert.

7. Verfahren zur Herstellung eines Gemisches aus dem C₁₅-Triarylphosphoniumsalz der allgemeinen Formel III und dem neuen isomeren der allgemeinen Formel iso-III 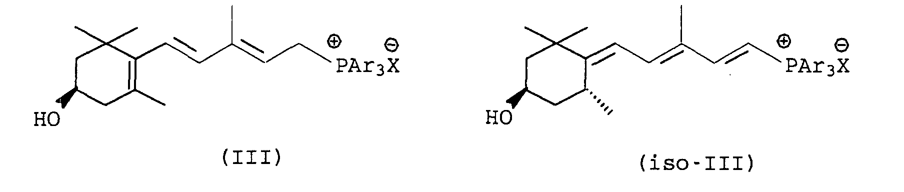 in der Ar für einen Arylrest, vorzugsweise einen ggf. substituierten Phenylrest steht und
X für ein Äquivalent eines Anions einer starken Säure, insbesondere für Cl, Br oder (HSO₄) steht,
dadurch gekennzeichnet, daß man das neue (4R,6R)-1-(3-Hydroxy-3-methyl-penta-1,4-dien-1-yl)-2,2,6-trimethyl-7-oxa-bicyclo[2.2.1]-heptan der Formel VI 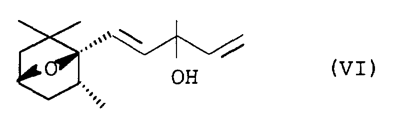 mit einem Triarylphosphin und einer starken Säure umsetzt.

8. [5-(4R,6R)-(4-Hydroxy-2,2,6-trimethyl-cyclohex-1-yliden)-3-methyl-penta-1,3-dienyl]-triphenylphosphoniumsalz der allgemeinen Formel iso-III 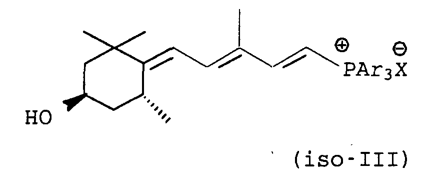 im Gemisch mit dem entsprechenden [5-(4R)-(4-Hydroxy-2,2-6-trimethyl-cyclohex-1-en-1-yl)-3-methyl-penta-2,4-dienyl]-triarylphosphoniumsalz der allgemeinen Formel III 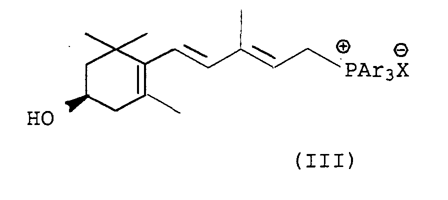 in der Ar für einen Arylrest, vorzugsweise einen gegebenenfalls substituierten Phenylrest steht und X für ein Äquivalent des Anions einer starken Säure steht.

9. Verfahren zur Herstellung von (3R, 3'R)-Zeaxanthin, dadurch gekennzeichnet, daß man ein neues [5-(4R,6R)-(4-Hydroxy-2,2,6-trimethyl-cyclohex-1-yliden)-3-methyl-penta-1,3-dienyl]triarylphosphoniumsalz der allgemeinen Formel iso-III oder ein Gemisch aus diesem neuen Triarylphosphoniumsalz der allgemeinen Formel iso-III und einem [5-(4R)-(4-Hydroxy-2,2,6-trimethyl-cyclohex-1-yl)-3-methyl-penta-1,3-dienyl] -triarylphosphoniumsalz der allgemeinen Formel III in einer doppelten Wittig-Reaktion mit 2,7-Dimethyl-octa-2,4,6-trien-l,8-dial umsetzt.

10. Verfahren gemäß Anspruch 2, dadurch gekennzeichnet, daß man das für Reaktionsstufe A. benötigte Dichlormethan-Lithium durch Metallierung von Dichlormethan mit Butyl-Lithium bei Temperaturen von -90 bis -60°C oder mit einem Lithiumdialkylamin bei -70 bis -30°C herstellt.

## Claims

1. A process for preparing (3R, 3'R)-zeaxanthin of the formula I 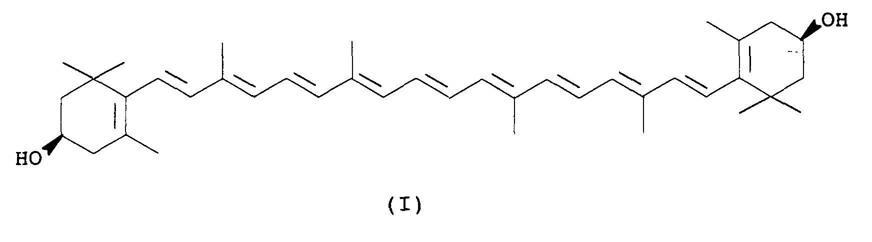 which comprises
A. (4R)-4-hydroxy-2,2,6-trimethylcyclohexanone of the formula II 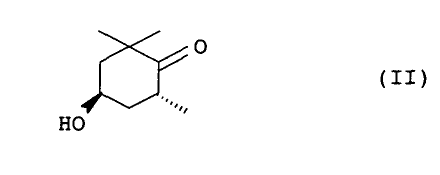 being reacted with dichloromethyl lithium in an inert solvent at from -120 to -40°C,
B. the novel (4R,6R)-1-formyl-2,2,6-trimethyl-7-oxabicyclo[2.2.1]heptane of the formula IV 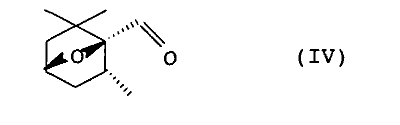 which is obtained on warming the reaction mixture to 20 to 60°C, being reacted either in isolated form or else directly in the form of the resulting reaction mixture with acetone or a dialkyl 2-oxopropylphosphonate,
C. the resulting novel (4R,6R)-1-(3-oxo-1-butenyl)-2,2,6-trimethyl-7-oxabicyclo[2.2.1]heptane of the formula V 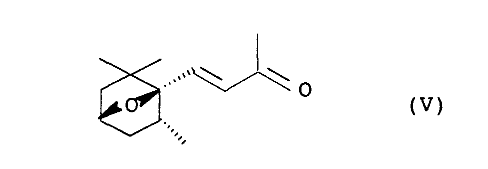 being converted in a conventional way by vinylation or ethynylation and subsequent partial hydrogenation into the novel (4R,6R)-1-(3-hydroxy-3-methyl-1,4-pentadienyl)-2,2,6-trimethyl-7-oxabicyclo[2.2.1]heptane of the formula VI 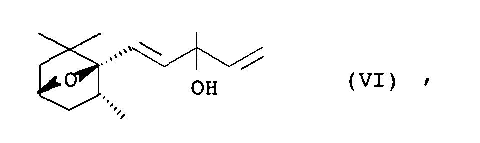
D. the latter being reacted with a triarylphosphine, preferably triphenylphosphine, and a strong acid to give a mixture of the C₁₅-triarylphosphonium salt of the formula III and the novel isomer of the formula iso-III 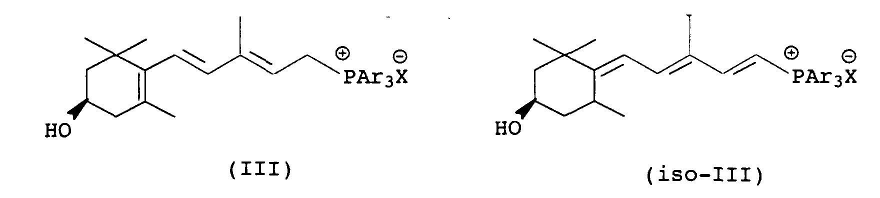 where Ar is an aryl radical, preferably an unsubstituted or substituted phenyl radical, and X is one equivalent of an anion of a strong acid, in particular Cl, Br or (HSO₄),
and
E. in each case about 2 mol of the resulting mixture of the C₁₅-triarylphosphonium salt III and the novel iso-III being converted by a double Wittig reaction with 2,7-dimethyl-2,4,6-octatrienedial of the formula VIII 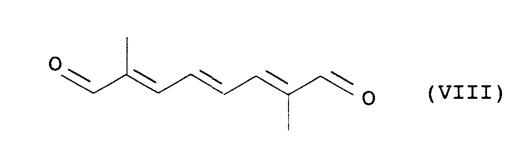 into zeaxanthin of the formula I.

2. A process for preparing the novel (4R,6R)-1-formyl-2,2,6-trimethyl-7-oxabicyclo[2.2.1]heptane of the formula IV 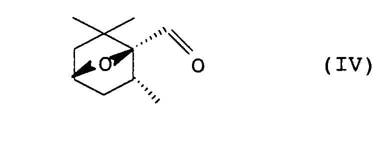 which comprises (4R)-4-hydroxy-2,2,6-trimethylcylohexanone of the formula II 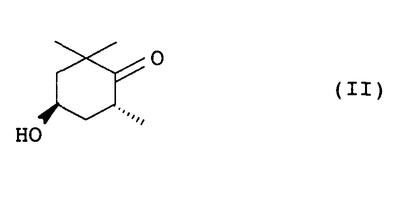 being reacted with dichloromethyllithium in an inert solvent at from -120 to -40°C, and the resulting reaction mixture being formed, in the presence or absence of an alkali metal alcoholate or an alkali metal hydroxide, to from 20 to 60°C.

3. (4R,6R)-1-Formyl-2,2,6-trimethyl-7-oxabicyclo[2.2.1]heptane of the formula IV

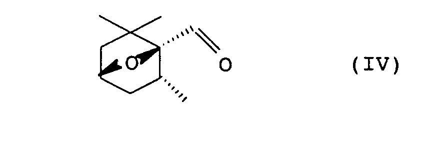

4. A process for preparing the novel (4R,6R)-1-(3-oxo-1-butenyl)-2,2,6-trimethyl-7-oxabicyclo[2.2.1]heptane of the formula V 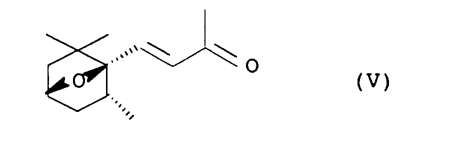 which comprises the novel (4R,6R)-1-formyl-2,2,6-trimethyl-7-oxabicyclo[2.2.1]heptane of the formula IV 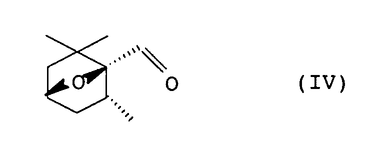 being reacted either in isolated form or directly in the form of the reaction mixture obtained on reacting (4R)-4-hydroxy-2,2,6-trimethylcyclohexanone of the formula II 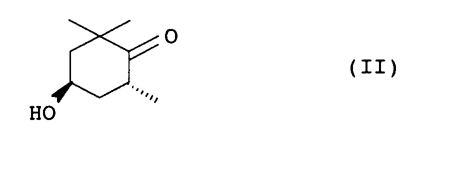 with dichloromethyllithium, which has been obtained by metallation of dichloromethane with alkyllithium compounds at from -120 to -70°C or with lithium dialkylamides at from -70°C to -40°C, in an inert solvent at from -120 to -40°C, and subsequently warming to 20 to 60°C, with acetone in an aldol condensation.

5. (4R,6R)-1-(3-Oxo-1-butenyl)-2,2,6-trimethyl-7-oxabicyclo[2.2.1]heptane of the formula V

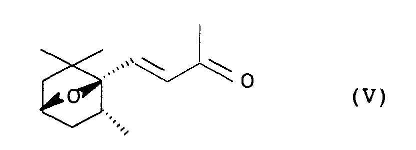

6. A process for preparing the novel (4R,6R)-1-(3-hydroxy-3-methyl-1,4-pentadienyl)-2,2,6-trimethyl-7-oxabicyclo[2.2.1]heptane of the formula VI 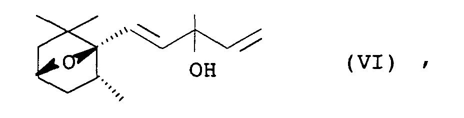 which comprises the novel (4R,6R)-1-(3-oxo-1-butenyl)-2,2,6-trimethyl-7-oxabicyclo[2.2.1]heptane of the formula V 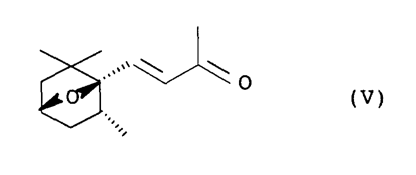 being, in a conventional way, vinylated or initially ethynylated and subsequently partially hydrogenated.

7. A process for preparing a mixture of the C₁₅-triarylphosphonium salt of the formula III and the novel isomer of the formula iso-III 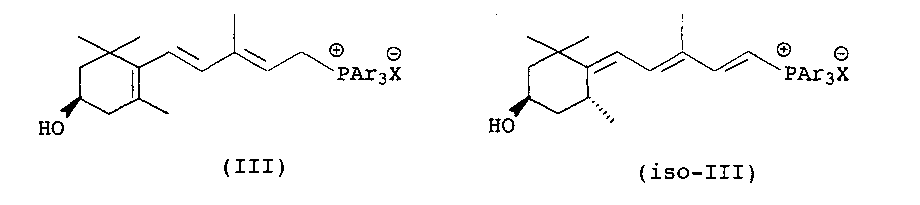 where Ar is an aryl radical, preferably an unsubstituted or substituted phenyl radical, and
X is one equivalent of an anion of a strong acid, in particular Cl, Br or (HSO₄),
which comprises the novel (4R,6R)-1-(3-hydroxy-3-methyl-1,4-pentadienyl)-2,2,6-trimethyl-7-oxabicyclo[2.2.1]heptane of the formula VI 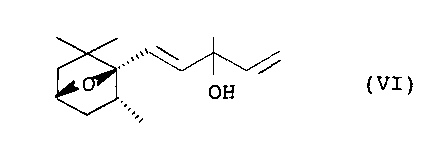 being reacted with a triarylphosphine and a strong acid.

8. [5-(4R,6R)-(4-Hydroxy-2,2,6-trimethyl-1-cyclohexylidene)-3-methyl-1,3-pentadienyl]triarylphosphonium salt of the formula iso-III 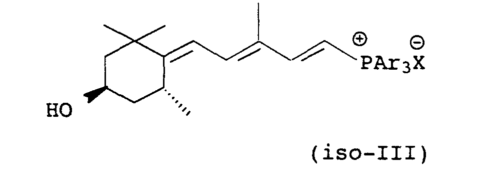 mixed with the corresponding [5-(4R)-(4-hydroxy-2,2,6-trimethyl-1-cyclohexenyl)-3-methyl-2,4-pentadienyl]triarylphosphonium salt of the formula III 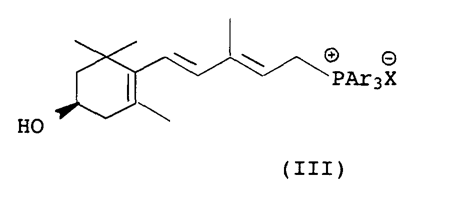 where Ar is an aryl radical, preferably an unsubstituted or substituted phenyl radical, and X is one equivalent of the anion of a strong acid.

9. A process for preparing (3R, 3'R)-zeaxanthin, which comprises a novel [5-(4R,6R)-(4-hydroxy-2,2,6-trimethyl-1-cyclohexylidene)-3-methyl-1,3-pentadienyl]triarylphosphonium salt of the formula iso-III or a mixture of this novel triarylphosphonium salt of the formula iso-III and a [5-(4R)-(4-hydroxy-2,2,6-trimethyl-1-cyclohexenyl)-3-methyl-2,4-pentadienyl]triarylphosphonium salt of the formula III being reacted in a double Wittig reaction with 2,7-dimethyl-2,4,6-octatrienedial.

10. A process as claimed in claim 2, wherein the dichloromethyllithium required for reaction stage A. is prepared by metallation of dichloromethane with butyllithium at from -90 to -60°C or with a lithium dialkylamide at from -70 to -30°C.

## Revendications

1. Procédé pour la préparation du (3R, 3'R)-zéaxanthène de formule générale I 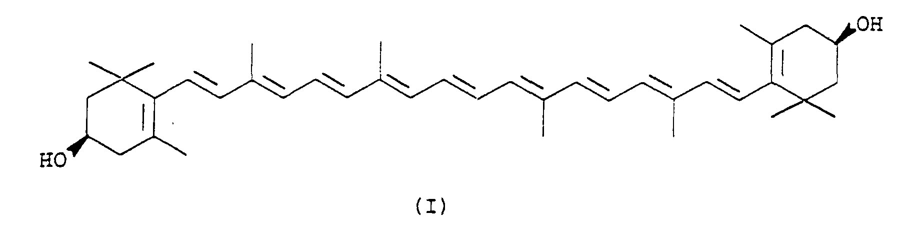 caractérisé par le fait que
A. on fait réagir la (4R)-4-hydroxy-2,2,6-triméthyl-cyclohexanone de formule II 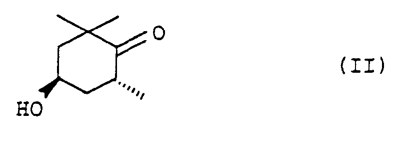 à des températures de -120 à -40°C, dans un solvant inerte, avec le dichlorométhyl-lithium,
B. le chauffage du mélange de réaction à des températures de 20 à 60°C donnant le nouveau (4R,6R)-1-formyl-2,2,6-triméthyl-7-oxa-bicyclo[2.2.1]heptane de formule IV 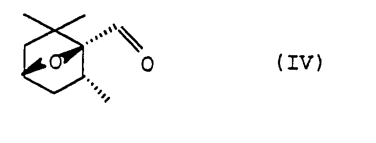 qu'on fait réagir, après l'avoir isolé ou directement sous la forme du mélange de réaction obtenu, avec l'acétone ou un (2-oxo-propyl)phosphate de dialkyle,
C. cette réaction donnant le nouveau (4R,6R)-1-(3-oxo-but-1-én-1-yl)-2,2,6-triméthyl-7-oxo-bicyclo[2.2.1]heptane de formule V 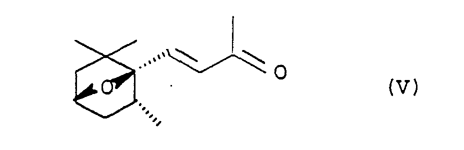 qu'on convertit de manière connue en soi, par vinylation ou éthynylation suivie d'une hydrogénation partielle, en le nouveau (4R, 6R)-1-(3-hydroxy-3 -méthyl-penta-1,4-dién-1-yl)-2,2,6-triméthyl-7-oxa-bicyclo[2.2.1]heptane de formule VI 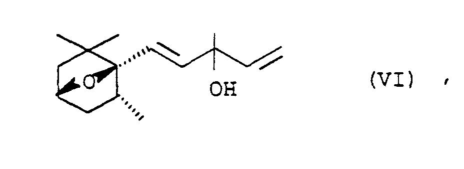
D. qu'on fait réagir avec une triarylphosphine, de préférence la triphénylphosphine et un acide fort, cette réaction donnant un mélange du sel de triarylphosphonium en C15 de formule générale III et du nouvel isomère de formule générale iso-III 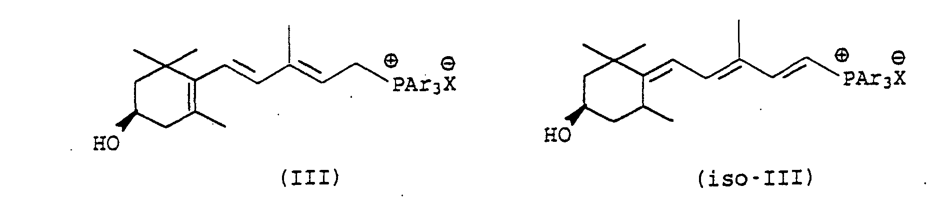 dans lesquelles Ar représente un radical aryle, de préférence un radical phényle éventuellement substitué et
X représente un équivalent d'un anion d'un acide fort, plus spécialement C1, Br ou (HSO₄),
et
E. on convertit environ 2 mol du mélange des sels de triarylphosphonium en C15 III et du nouvel iso-III par une double réaction d'oléfination de Wittig avec environ 2 mol du 2,7-diméthyl-octa-2,4,6-triène-1,8-dial de formule VIII 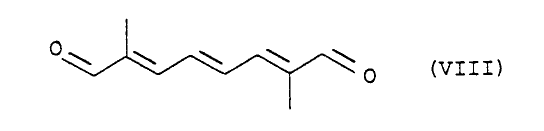 en le zéaxanthène de formule I.

2. Procédé de préparation du nouveau (4R,6R)-1-formyl-2,2,6-formyl-2,2,6-triméthyl-7-oxa-bicyclo[2.2.1]heptane de formule IV 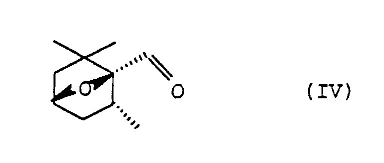 caractérisé par le fait que l'on fait réagir la (4R)-hydroxy-2,2,6-triméthyl-cyclohexanone de formule II 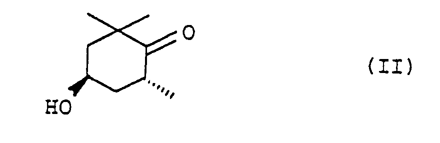 à des températures de -120 à -40°C, dans un solvant inerte, avec le dichlorométhyllithium puis on chauffe le mélange de réaction obtenu, éventuellement en présence d'un alcoolate alcalin ou d'un hydroxyde alcalin, à des températures de 20 à 60°C.

3. Le (4R,6R)-1-formyl-2,2,6-triméthyl-7-oxa-bicyclo[2.2.1]-heptane de formule IV

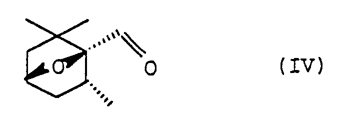

4. Procédé de préparation du nouveau (4R,6R)-1-(3-oxo-but-1-én-1-yl)-2,2,6-triméthyl-7-oxa-bicyclo[2.2.1]heptane de formule V 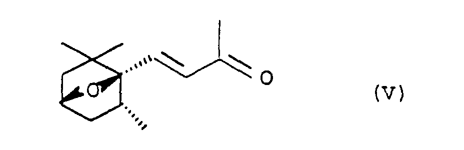 caractérisé par le fait que l'on fait réagir à des températures de -120 à -40°C, dans un solvant inerte, le nouveau (4R,6R)-1-formyl-2,2,6-triméthyl-7-oxa-bicyclo[2.2.1]heptane de formule IV 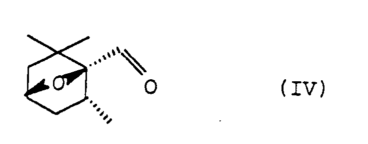 soit après l'avoir isolé, soit directement sous la forme du mélange de réaction obtenu par réaction de la (4R)-4-hydroxy-2,2,6-triméthylcyclohexanone de formule II 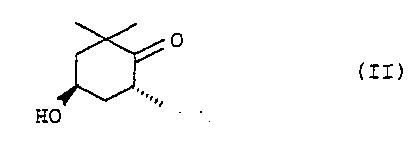 avec le dichlorométhyl-lithium, lui-même obtenu par métallisation du dichlorométhane à l'aide de dérivés d'alkyllithium à des températures de -120 à -70°C ou à l'aide de lithium-dialkylamines à des températures de -70 à -40°C, en faisant suivre d'un chauffage à des températures de 20 à 60°C et d'une condensation aldolique avec l'acétone.

5. Le (4R,6R)-1-(3-oxo-but-1-én-1-yl)-2,2,6-triméthyl-7-oxa-bicyclo[2.2.1]heptane de formule V

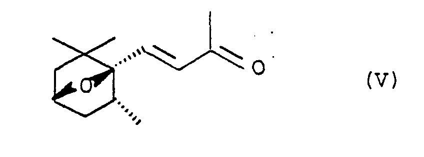

6. Procédé de préparation du nouveau (4R,6R)-1-(3-hydroxy-3-méthyl-penta-1,4-dién-1-yl)-2,2,6-triméthyl-7-oxa-bicyclo[2.2.1]heptane de formule VI 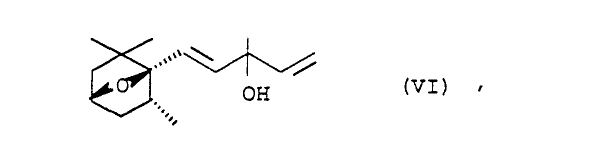 caractérisé par le fait que l'on soumet le nouveau (4R,6R)-1-(3-oxo-but-1-én-1-yl)-2,2,6-triméthyl-7-oxa-bicyclo[2.2.1]heptane de formule V 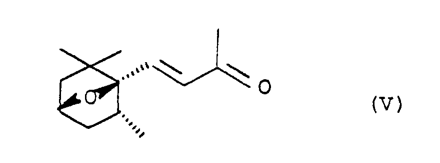 à vinylation ou d'abord à éthynylation puis hydrogénation partielle, de manière connue en soi.

7. Procédé pour la préparation d'un mélange du sel de triarylphosphonium en C15 de formule générale III et d'un nouvel isomère de formule générale iso-III 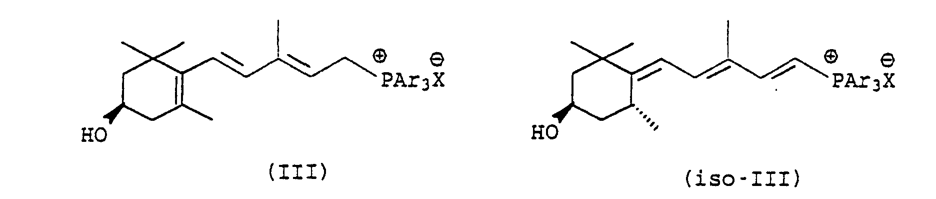 dans lesquelles Ar représente un radical aryle, de préférence un radical phényle éventuellement substitué et
X représente un équivalent d'un anion d'un acide fort, plus spécialement Cl, Br ou (HSO₄),
caractérisé par le fait que l'on fait réagir le nouveau (4R,6R)-1-(3-hydroxy-3-méthyl-penta-1,4-dién-1-yl)-2,2,6-triméthyl-7-oxa-bicyclo-[2.2.1]heptane de formule VI 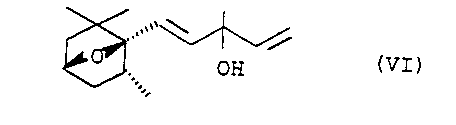 avec une triarylphosphine et un acide fort.

8. Le sel de [5-(4R,6R)-(4-hydroxy-2,2,6-triméthyl-cyclohex-1-ylidène)-3-méthylpenta-1,3-diényl]triphénylphosphonium de formule générale iso-III 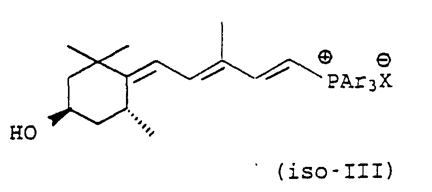 en mélange avec le sel de [5-(4R)-(4-hydroxy-2,2,6-triméthyl-cyclohex-1-én-1-yl)-3-méthyl-penta-2,4-diényl]triarylphosphonium correspondant de formule générale III 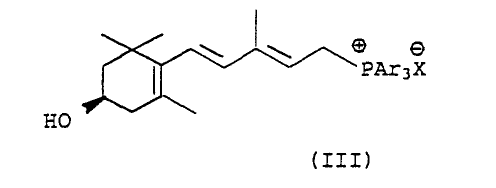 dans lesquelles Ar représente un radical aryle, de préférence un radical phényle éventuellement substitué et X représente un équivalent de l'anion d'un acide fort.

9. Procédé de préparation du (3R,3'R)-zéaxanthène caractérisé par le fait que l'on fait réagir un nouveau sel de [5-(4R,6R)-(4-hydroxy-2,2,6-triméthyl-cyclohex-1-ylidène)-3-méthyl-penta-1,3-diényl]triarylphosphonium de formule générale iso-III ou un mélange de ce nouveau sel de triarylphosphonium de formule générale iso-III et d'un sel de (5-(4R)-(4-hydroxy-2,2,6-triméthyl-cyclohex-1-yl)-3-méthyl-penta-1,3-diényl]triarylphosphonium de formule générale III dans une double réaction de Wittig avec le 2,7-diméthyl-oxa-2,4,6-triène-1,8-dial.

10. Procédé selon la revendication 2, caractérisé par le fait que l'on prépare le dichlorométhane-lithium, nécessaire pour le stade de réaction A. par métallisation du dichlorométhane à l'aide du butyl-lithium à des températures de -90 à -60°C ou à l'aide d'une lithium-dialkylamine à des températures de -70 à -30°C.
